# EUROPÄISCHE PATENTSCHRIFT

(11) **EP 0 609 336 B1**
(45) Veröffentlichungstag und Bekanntmachung des Hinweises auf die Patenterteilung: **28.01.1998**
(21) Anmeldenummer: 92922216.4
(22) Anmeldetag: 22.10.1992
(51) Int. Cl.: A61K 31/557, A61K 49/04, A61K 49/00

(54) **VERWENDUNG VON PROSTACYCLIN-DERIVATEN ZUR VERHINDERUNG ODER BEHANDLUNG VON STÖRUNGEN DER MIKROZIRKULATION BEI GABE VON RÖNTGEN-, NMR- ODER ULTRASCHALLKONTRASTMITTELN**
USE OF PROSTACYCLIN DERIVATIVES TO PREVENT OR TREAT DISORDERS OF THE MICROCIRCULATING SYSTEM WHEN X-RAY, NMR OR ULTRASONIC CONTRASTING AGENTS ARE ADMINISTERED
UTILISATION DE DERIVES DE LA PROSTACYCLINE POUR L'INHIBITION OU LE TRAITEMENT DE TROUBLES DE LA MICROCIRCULATION LORS DE L'UTILISATION D'AGENTS DE CONTRASTE DANS DES PROCEDES FAISANT APPEL AUX RAYONS X, A LA RMN OU AUX ULTRASONS

(30) Priorität: 22.10.1991 DE 4135193
(43) Veröffentlichungstag der Anmeldung: 10.08.1994
(73) Patentinhaber: SCHERING AKTIENGESELLSCHAFT, 13353 Berlin (DE)
(72) Erfinder: KLOPP, Rainer, D-10409 Berlin (DE); NIEMER, Wolfgang, D-10245 Berlin (DE); SCHIPPEL, Wolfgang, D-10247 Berlin (DE); KRAUSE, Werner, D-13505 Berlin (DE)
(86) Internationale Anmeldenummer: DE9200904
(87) Internationale Veröffentlichungsnummer: WO9307875

(56) Entgegenhaltungen:
- EP-A- 0 217 419
- EP-A- 0 385 859
- EP-A- 0 407 148
- WO-A-86/00808
- WO-A-89/04828
- CHEMICAL ABSTRACTS, vol. 100, no. 13 Columbus, Ohio, US; abstract no. 97374u,
- EUR. J. CLIN. PHARMACOL. Bd. 41, Nr. 2, 1991, GERMANY Seiten 131 - 136 L. CASPARY 'Intraveneous infusion of iloprost in arterial occlusive disease: dose-dependent effects on skin microcirculation.'
- J. AM. COLL. CARDIOL. Bd. 16, Nr. 4, Oktober 1990, U.S.A. Seiten 770 - 778 CW. ABBOTTSMITH 'Fate of patients with acte myocardial infarction with patency of the infarct-related vessel achieved with successful thrombolysis versus rescue angioplasty.'

## Beschreibung

Kontrastmittel sind unerläßliche Hilfsmittel in der medizinischen Diagnostik. Sie ermöglichen immer stärkeren Kontrast in den erhaltenen Aufnahmen sowie besseres Erkennen der verschiedenen Gewebe und Organe. Unter anderem finden sie in Röntgen-, Ultraschall- und magnetischen Resonanz-Verfahren ihre Anwendung. Diesem sehr nützlichen Anwendungsbereich stehen eine Vielzahl von unerwünschten Nebenwirkungen wie zum Beispiel Veränderungen an Endothelzellen, Schädigung der Erythrozyten, Beeinflussung des Blutgerinnungssystems, Störung der Mikrozirkulation bis hin zur vollständigen Hämostase, Verstärkung von bereits vorhandener Störung der Mikrozirkulation im pathogenen Gewebe, Veränderungen der BlutHirnSchranke oder negative Beeinflussung des Blutdrucks gegenüber. Eine besondere Bedeutung kommt der Störung der Mikrozirkulation zu. Die Mikrozirkulation ist funktionell der wichtigste Teil des Kreislaufsystems. Im Bereich der Mikrozirkulation findet der Stoffaustausch statt. Bei Störung der Mikrozirkulation sind unter anderem Abnahme der blutzellperfundierten Knotenpunkte, Zunahme von Adhäsionen der Blutzellen an der Venoleninnenwand und Erythrozytenaggregation zu beobachten. Die arterioläre Vasomotion wird ebenfalls beeinträchtigt. Eine Reihe von Erkrankungen, nicht nur des Herz-Kreislauf-Systems, sind auf Störungen der Mikrozirkulation zurückzuführen. Daher ist es unbedingt erstrebenswert, eine Störung der Mikrozirkulation als Auswirkung einer Kontrastmittelgabe zu vermeiden oder sofort zu behandeln.

EP-A-0 217 419 offenbart eine Zusammensetzung zur Verhinderung von Störungen bei der Durchführung von Angioplastie, enthaltend Prostacyclin und ein angiographisches Kontrastmittel.

EP-A-0 385 859 beschreibt eine Zusammensetzung zur Inhibierung oder Verzögerung des Wachstums von malignen Krebszellen, enthaltend Prostacyclin und ein radiotherapeutisches Mittel.

Von chemisch stabilen Prostacyclinderivaten sind eine Vielzahl von pharmakologischen Wirkungen bereits bekannt.

Überraschend wurde nun gefunden, daß Prostacyclinderivate bei Verabreichung kurz vor oder nach einer Gabe von Röntgen-, Ultraschall- oder NMR-Kontrastmitteln oder bei gemeinsamer Verabreichung mit den genannten Kontrastmitteln in signifikantem Maße die Mikrozirkulation erhalten oder wiederherstellen.

Die Erfindung betrifft die Verwendung eines Prostacyclinderivates oder des entsprechenden β-Cyclodextrin-Clathrates oder der mit Liposomen verkapselten Form zur Herstellung eines Arzneimittels zur Verhinderung oder Behandlung von Störungen der Mikrozirkulation bei Gabe von Röntgen-, NMR- oder Ultraschallkontrastmitteln.

Bevorzugt betrifft die vorliegende Erfindung die Verwendung eines oder mehrerer Prostacyclin-Derivate der allgemeinen Formel I 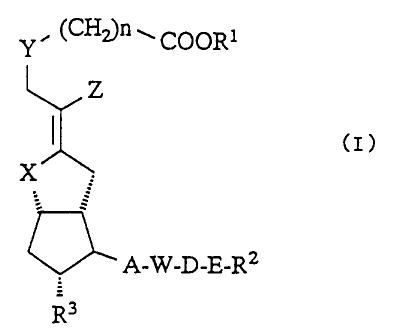 worin
R¹ Wasserstoff oder ein C₁-C₄-Alkylrest,
n 0 bis 3,
X,Y unabhängig voneinander eine -CH₂ -Gruppe oder ein Sauerstoffatom,
Z Wasserstoff, Fluor oder CN,
A eine trans -CH=CH- oder eine -C=C-Gruppe,
W eine freie oder an der Hydroxygruppe funktionell abgewandelte Hydroxymethylengruppe, wobei die Hydroxygruppe α- oder β-ständig sein kann,
D eine gradkettige oder verzweigte gesättigte C₁-C₅-Alkylengruppe
E eine -C=C-Gruppe
R² eine C₁-C₂-Alkylgruppe
R³ eine freie oder funktionell abgewandelte Hydroxygruppe bedeuten, und falls R¹ Wasserstoff bedeutet,
deren Salze mit physiologisch verträglichen Basen, sowie deren α, β- oder γ-Cyclodextrin-Clathrate sowie deren mit Liposomen verkapselte Form oder Ataprost, Beraprost, BW-15AU, Ciprosten, CS 570, FCE 22509, Naxaprosten, RS-93427, SC 39902 oder Taprosten zur Herstellung eines Arzneimittels zur Verhinderung oder Behandlung von Störungen der Mikrozirkulation bei der Gabe von Röntgen-, Ultraschall- oder NMR-Kontrastmitteln.

Besonders bevorzugt betrifft die vorliegende Erfindung die Verwendung der Prostacyclin-Derivate Iloprost, Iloprost-Clathrat, Cicaprost, Cicaprost-Clathrat, Eptaloprost oder Eptaloprost-Clathrat.

Als Alkylgruppen in R¹ sind grad- oder verzweigtkettige Alkylgruppen mit 1-4 C-Atomen zu betrachten wie beispielsweise Methyl, Ethyl, Propyl, Isopropyl, Butyl, Isobutyl, tert.-Butyl. Die Alkylgruppen R¹ können gegebenenfalls substituiert sein durch Halogenatome, Methoxy, Ethoxy, Phenyl oder (C₁-C₂)-Dialkylamine.

Als Substituenten seien beispielsweise genannt Fluor-, Chlor- oder Bromatome, Phenyl, Dimethylamin, Diethylamin, Methoxy oder Ethoxy. Bevorzugte Alkylgruppen R¹ sind Methyl, Ethyl, Dimethylaminopropyl.

Als Alkylgruppe R² seien Methyl und Ethyl genannt.

Die Hydroxygruppen in R³ und W können als freie Hydroxygruppen vorliegen, wobei die Hydroxygruppe in W bevorzugt α-ständig ist, oder funktionell abgewandelt sein kann, beispielsweise durch Veretherung oder Veresterung. Freie Hydroxygruppen werden bevorzugt. Als Ether- oder Acylreste kommen die dem Fachmann bekannten Reste in Betracht. Bevorzugt sind leicht spaltbare Etherreste wie beispielsweise Tetrahydropyranyl, Tetrahydrofuranyl, α-Ethoxyethyl, Trimethylsilyl, Dimethyl-tert.-butylsilyl, Diphenyl-tert.-butylsilyl oder Tribenzylsilyl.
Als Acylreste seien beispielsweise genannt Acetyl, Propionyl, Butyryl oder Benzoyl.

Als Alkylengruppe D kommen geradkettige oder verzweigte gesättigte Alkylgruppen mit 1-5 C-Atomen in Betracht, beispielsweise Methylen, Ethylen, 1- oder 2-Propylen, Ethylethylen, Trimethylen, Tetramethylen, Pentamethylen, 1-Methyldimethylen, 1-Methyltrimethylen, 1-Methyltetramethylen.

Zur Salzbildung mit den freien Säuren (R¹=H) sind anorganische und organische Basen geeignet, wie sie dem Fachmann zur Bildung physiologisch verträglicher Salze bekannt sind. Beispielsweise seien genannt: Alkalihydroxide wie Natriumoder Kaliumhydroxid, Erdalkalihydroxide wie Calciumhydroxid, Ammoniak, Amine wie Ethanolamin, Diethanolamin, Triethanolamin, N-Methylglucamin, Morpholin, Tris(hydroxymethyl)methylamin usw.

Die Clathrate mit α-, β- oder γ-Cyclodextrin werden analog der Vorschrift WO 87/05294 erhalten. Bevorzugte Clathrate sind die mit β-Cyclodextrin.

Liposomen werden z.B. nach dem in "Pharmazie in unserer Zeit 11, 98 (1982)" beschriebenen Verfahren hergestellt.
Die Herstellung der Verbindungen der Formel I wird detailliert in EP 2234 B1
und EP 11591 B1 beschrieben.

In EP 11591 B1 werden für Prostacyclinderivate der Formel I folgende pharmakologische Eigenschaften beschrieben:
Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose. Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse oder Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Prostaglandinanaloga antiproliferative Eigenschaften.
In EP 86404 B1 wird die Anwendung von Carbacyclinen zur Prophylaxe und Therapie ischaemischer Attacken des ZNS-Systems, zur Zytoprotektion in der Leber und im Pankreas sowie die Kombination mit β-Blockern oder Diuretika beschrieben. Aus WO 86/00808 ist die Zytoprotektion der Niere sowie die Eignung der Prostacyclinderivate der Formel I zur Behandlung von zu transplantierenden Organen bekannt. In DE 35 26 362 A1 wird die Kombination der Prostacyclinderivate der Formel I mit Thromboxanantagonisten zur Anwendung bei thrombotischen oder thromboembolischen Krankheitsbildern beschrieben.

Aus DE 35 44 663 A1 ist die Kombination der Prostacyclinderivate der Formel I mit Fibrinolytika zur Verhinderung von Rethrombosen nach einer Thrombose bekannt.
In DE 36 08 088 A1 sind die Clathrate der Carbacyclinderivate der Formel I beschrieben. Aus DE 36 31 169 A1 ist zusätzlich zu den in EP 11591 B1 beschriebenen Verabreichungsformen die topische Verabreichurlgsform bekannt.

Die Verwendung der Prostacyclinderivate der Formel I oder des entsprechenden β-Cyclodextrinclathrats oder der mit Liposomen verkapselten Form zur Herstellung eines Arzneimittels zur Verhinderung oder Behandlung von Störungen der Mikrozirculation bei der Gabe von Röntgen-, MNR- oder Ultrschalkontrastmitteln ist in keiner der vorstehenden Offenlegungsschriften oder Patente genannt.

Völlig überraschend haben Untersuchungen am Tiermodell gezeigt, daß sich die Prostacyclinderivate der Formel I bei der Behandlung bzw. Vermeidung von Störungen der Mikrozirkulation durch Gabe von Röntgen-, Ultraschall- oder NMR-Kontrastmitteln erfolgreich verwenden lassen.

Die bei der Anwendung eines Kontrastmittels im Tierversuch beobachteten Störungen der Mikrozirkulation wie z.B. drastische Reduktion der blutzellperfundierten Knotenpunkte im mikrovaskulären Netzwerk, Zunahme aggregierter Erythrozyten, vermehrte Adhäsionen von weißen Blutzellen an der Venoleninnenwand sowie Beeinträchtigung der arteriölaren Vasomotion sind in der 2. und 7. Minute nach der Kontrastmittelgabe besonders ausgeprägt, was mit den klinischen Erfahrungen zu Nebenwirkungen von und Zwischenfällen mit Kontrastmitteln (sogenannte Sofortreaktion nach ca. 2 min. bzw. sogenannte Spätreaktion nach ca. 7 min.) koinzidiert.

Bei der Gabe von partikelhaltigen Kontrastmitteln, wie sie bei einigen Ultraschallkontrastmiteln oder NMR-Kontrastmitteln vorkommen, erscheint selbst dann eine Gabe der Prostacyclinderivate der Formel I zur Behandlung oder Vermeidung von Störungen der Mikrozirkulation sinnvoll, wenn die bei Untersuchung an gesundem Gewebe beobachtete Störung der Mikrozirkulation relativ gering ist. Denn es darf nicht übersehen werden, daß bei gefäßpathologischen Zuständen, und nur dann erfolgt im allgemeinen eine Untersuchung mit Kontrastmittelgabe, die in die Mikrozirkulation gefluteten Kontrastmittelpartikel bestehende Störungen sogar verstärken könnten.

Die Applikation der Prostacyclinderivate der Formel I vor -, gleichzeitig mit - und nach Kontrastmittelgabe bewirkt eine weitgehende Vermeidung bzw. eine entschieden verringerte Störung der Mikrozirkulation verbunden mit einer schnelleren Wiederherstellung der Mikroperfusion.

Die Prostacyclinderivate der Formel I können topisch oder intravasal (i.a., i.v.) appliziert werden. Das Kontrastmittel wird intravasal (i.a., i.v.) verabfolgt.
Die Dosis der Prostacyclin-Derivate beträgt 1,6-0,0004 µg/kg Körpermasse, bevorzugt 1,2-0,0004 µg/kg Körpermasse.

Für die Kombination sind Röntgen-, Ultraschall- und NMR-Kontrastmittel geeignet.

Bevorzugte Kontrastmittel sind Iotrolan, Iopromid, Iohexol, Iosimid, Metrizamid, Salze von Amidoessigsäure, Iotroxinsäure, Iopamidol, 5-Hydroxyacetamido-2,4,6-triiodo-isophthalsaure-(2,3-dihydroxy-N-methylpropyl)(2hydroxyethyl)-diamid, 3-Carbamoyl-5-[N-[2-hydroxyethyl)-acetamido]-2,4,6-triiodo-benzoesäure[(1RS,2SR)-2,3-dihydroxy-1-hydroxymethylpropyl]amid, Dispersionen von Iodipamidethylester, Gadolinium DTPA, Gadolinium DOTA, der Gadoliniumkomplex von 10[1-Hydroxymethyl-2,3-dihydroxypropyl]-1,4,7-tris(carboxymethyl)-1,4,7,10-tetraazacyclododecan], Pro Hance ^{R}, Magnevist ^{R}, Omniscan ^{R}, Eisen-oder Manganporphyrinchelate, stabile Magnetitdispersionen, Dispersionen von Galaktosemikropartikeln mit oder ohne Additive in Wasser, eine Galaktoselösung oder Dispersionen von Mikrokugeln von eingeschlossener Luft, insbesondere Cyanacrylate oder Albuminmikiokugeln, Echovist ^{R}, Levovist ^{R}.

Die Dosiseinheit der Kontrastmittel kann 1-350 mg Jod/l für Röntgenkontrastmittel, 1-1000µg Partikel/kg Körpergewicht für Ultraschallkontrastmittel und 0,01-0,5^{mol} für NMR-Kontrastmittel betragen. Diese Angaben können jedoch nur Richtwerte sein, da die Kontrastmitteldosis stark von der gewünschten Anwendung abhängig ist.

Für die Kombination sind Prostacyclin-Derivate, insbesondere die unter Anspruch 2 genannten, geeignet.

Die Dosis der Prostacyclin-Derivate für die Kombination liegt im bereits für die getrennte Gabe angegeben Bereich.

Sollten die physikalischen Eigenschaften der Kontrastmittellosungen bzw. -dispersionen die Stabilität des Prostacyclin-Derivates über längere Zeit nicht gewährleisten, so wird für diese Fälle die Kombination in getrennten Dosiseinheiten bevorzugt.

### Bezugszeichen

### Figur 1

Darstellung der prozentualen Abnahme der blutzellperfundierten Knotenpunkte im mikrovaskulären Netzwerk im Vergleich zu den Ausgangsbedingungen
1 - Tiergruppe 1, Diatrizoat-Bolus
2 - Tiergruppe 2, 5 min nach Diatrizoat-Gabe Iloprost-Injektion
t - Zeitangabe in [min]
% - Blutzellperfundierte Knotenpunkte

### Figur 2

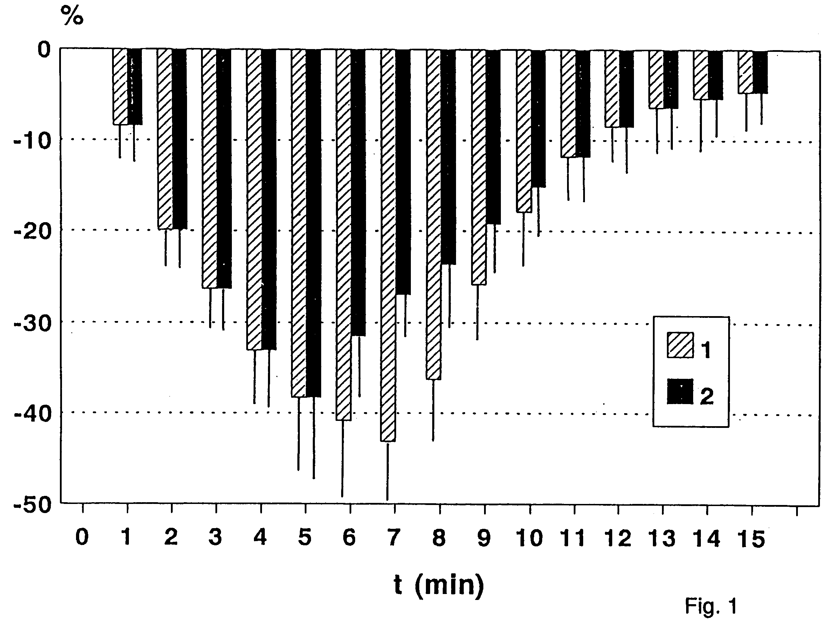

Darstellung der prozentualen Zunahme des Anteils aggregierter Erythrozyten an der Gesamtzellzahl im mikrovaskulären Netzwerk im Vergleich zu den Ausgangsbedingungen
1, 2, t - siehe Figur 1
% - Anteil aggregierter Erythrozyten in [%]

### Figur 3

Darstellung der Anzahl adhärierender Blutzellen an einer definierten Venoleninnenwandfläche
1, 2, t - siehe Figur 1
BZ - Anzahl der Blutzellen
A - definierte Venoleninnenwandfläche

### Figur 4, 5 und 6

Darstellung von Frequenzspektren der arteriolären Vasomotion.

### Beispiel 1

An narkotisierten Ratten des Wistar-Schönwalde-Stammes (Narkose mit UrethanChloralose-Gemisch - i.p., im. - , 10% Urethan in isotonischer NaCl-Lösung, 2% α-Chloralose in isotonischer NaCl-Lösung; 0,6 ml/100g Körpermasse als Initialdosis, 0,2 ml/100g Körpermasse als Erhaltungsdosis bei Bedarf) wird ein Mikrokatheder zur intraarteriellen Injektion des Kontrastmittels von der A. carotis communis bis zum Aortenbogen vorgeschoben. Es erfolgt einmalige Injektion eines körperwarmen Bolus Amidotrizoat/Urografin mit 600 mg J/kg Körpermasse. Die Injektionszeit beträgt 30s. Die Applikation von Iloprost erfolgt 5 min. nach Kontrastmittelgabe, bei intraarterieller Applikation auf demselben Injektionsweg wie das Kontrastmittel, bei intravenöser Applikation via V. femoralis und bei topischer Applikation via Intestinum/Mesenterium. Die Iloprost Dosis beträgt 0,8-1,6 µg Iloprost/kg Körpermasse in 2,0 ml Lösung/kg Körpermasse. Bei topischer Applikation werden 3 Tropfen dieser Lösung verwendet. Die Zahl der Versuchstiere beträgt bei intraarterieller Applikation 45, bei intravenöser Applikation 25 und bei topischer Applikation 20. Die Kontrolltiere erhalten physiologische Kochsalzlösung in der entsprechenden Applikation. Die Zahl der Kontrolltiere beträgt bei intraarterieller und intravenöser Applikation 12 und bei topischer Applikation 20.

### Ergebnisse

Die Befunderhebung erfolgt am narkotisierten Tier unter konstanten Randbedingungen durch kombinierte Auflicht-Durchlicht-Intravitalmikroskopie am exponierten Mesenterium/Intestinum in thermostatisierter Badlösung. Die Aufnahme der Daten erfolgt üblicherweise in einem Intervall von 5 min. vor der Kontrastmittelgabe bis 15 min. nach der Kontrastmittelgabe jede Minute.

Die blutzellperfundierten Knotenpunkte der intestinalen Mikroströmung nehmen durch Gabe von Iloprost zu. Der Vergleich mit der unbehandelten Kontrolle zeigt, daß Iloprost die in dem klinisch bedeutsamen Zeitintervall der 5. bis 10. Minute p.a. Amidotrizoat kontrastmittelinduzierten Störungen der Mikrozirkulation deutlich dämpft und daß eine schnellere Wiederherstellung der physiologischen Mikroperfusion erreicht wird.
Figur 1 zeigt die prozentuale Abnahme der bluttzellperfundierten Knotenpunkte im mikrovaskulären Netzwerk des Mesenteriums bei Kontrastmittelgabe im Vergleich zu den Ausgangsbedingungen sowie die Auswirkung der Iloprost-Gabe. Es werden zwei Tiergruppen verglichen; Gruppe 1 hat zum Zeitpunkt t=0 einen Diatrizoat-Bolus erhalten; Gruppe 2 erhielt nach dem Diatrizoat-Bolus zum Zeitpunkt t=0 einen Iloprost-Bolus von 0.8 µg/kg zum Zeitpunkt t=5. Die Balkendarstellung gibt Mittelwerte x. die aufgesetzten Linien die Standardabweichung S an.

Der prozentuale Anteil aggregierter Erythrozyten an der Gesamtzellzahl im mesenteralen Netzwerk wird durch Iloprost stark gesenkt. d.h. die Abnahme der Anzahl aggregierter Erythrozyten beschleunigt.

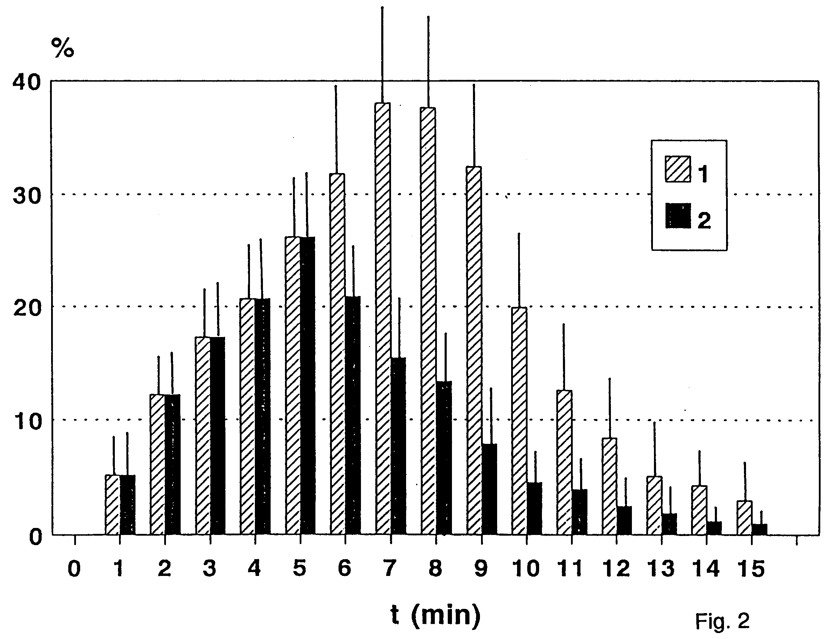

Figur 2 zeigt die prozentuale Zunahme des Anteils aggregierter Erythrozyten an der Gesamtzellzahl im mikrovaskulären Netzwerk des Mesenteriums bei Kontrastmittelgabe im Vergleich zu den Ausgangsbedingungen sowie die Auswirkung der Iloprost-Gabe. Die Tiergruppe 1 und 2 wurden wie im vorstehenden Versuch behandelt. Die Darstellung der Mittelwerte bzw. Standardabweichung erfolgt wie in Figur 1.

Die Abnahme der Anzahl adhärierender weißer Blutzellen an der Venoleninnenwand wie durch Iloprost stark beschleunigt.

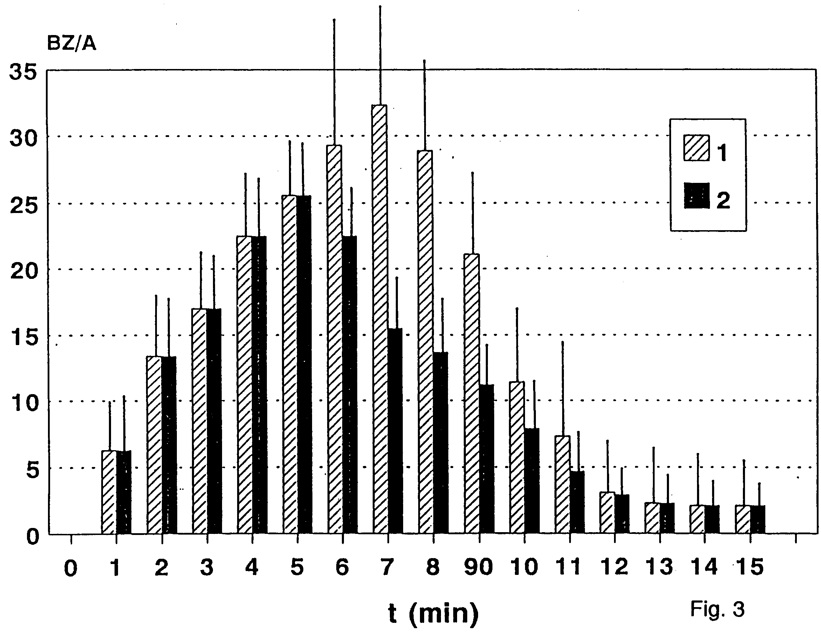

Figur 3 zeigt die Anzahl adhärierender Blutzellen an einer definierten Venoleninnenwandfläche des Mesenteriums in Abhängigkeit von der Zeit. Die Tiergruppe 1 und 2 wurden wie im vorstehenden Versuch behandelt. Die Darstellung der Mittelwerte bzw. Standardabweichung erfolgt wie in Figur 1.

### Beispiel 2

An narkotisierten adulten Ratten [Körpermasse X=304,7g (S=35,4g)] des WistarStammes (Narkose mit UfethanChloralose-Gemisch - i.p., i.m. - , 10% Urethan in isotonischer NaCl-Losung, 2% α-Chloralose in isotonischer NaCl-Lösung: 0,6 ml/ 100g Körpermasse als Initialdosis, 0,2 ml/ 100g Korpermasse als Erhaltungsdosis bei Bedarf) wird ein Mikrokatheder zur intravasalen Injektion das Kontrastmittels entweder in der A. carotis comm. bis zum Aortenbogen vorgeschoben (intraarterielle Injektion) oder in die V. femoralis eingeführt (intravenöse Injektion). Es erfolgt einmalige Injektion eines körperwarmen Bolus Diatrizoat/Urografin mit 600 mg J/kg Körpermasse. Die Injektionszeit betragt 30s. Die Applikation von Iloprost erfolgt 5 min. nach Kontrastmittelgabe, bei intravasaler Applikation auf gleichem Injektionsweg wie das Kontrastmittel oder bei topischer Applikation via Intestinum/Mesenterium. Die Iloprost Dosis beträgt 1,6-0,0004 µg Iloprost/kg Körpermasse in 2,0 ml Lösung/kg Körpermasse. Bei to-pischer Applikation werden 3 Tropfen dieser Lösung verwendet. Als Vergleichssubstanz wird physiologische Kochsalzlösung mit entsprechendem Volumen appliziert. Aufgrund der geringen Unterschiede der Körpermassen der Versuchstiere kann bei allen Versuchstieren ein nahezu gleiches Bolusvolumen von ca. 1 ml appliziert werden. Die Zahl der Versuchstiere beträgt 10 je Behandlungsgruppe.

Folgende Meßgrößen (Merkmale) werden bestimmt:
- Anzahl der aktuell zellperfundierten Knotenpunkte im mikrovaskularen Netzwerk ( %, prozentuale Änderung der Anzahl im Vergleich zu den Ausgangsbedingungen );
- Anzahl aggreglerter Erythrozyten im mikrovaskulären Strembett ( %, prozentualer Anteil aggregierter Erythrozyten an der Gesamtzellzahl im beobachteten Strombett; als Erythrozytenaggregation wird ein längeres Aneinanderhaften von mindestens 2 bis 3 Erythrozyten beobachtet, wobei die unterschiedlichen Arten der Aggregation nicht differenziert werden );
- Anzahl adhärierender Blutzellen an der Venoleninnenwand ( BZ/A; Anzahl der Blutzellen, die länger als 5 sec am Venolenendothel anhaften - bezogen auf eine definierte Venoleninnenwandfläche A 18000µ², die bei einem Venolendurchmesser dᵥ = 40µm durch eine axiale Venolenlänge lᵥ = 140µ gegeben ist).
- Arterioläre Vasomotion (Bestimmung des Innendurchmessers einer Arteriole mit einem Durchmesser von ca. 45 µm an einem bestimmten Ort zu jeder Sekunde im ca. 15 minütigen Beobachtungsintervall; Ermittlung des Ampliduden-Frequenz-Spektrums der Vasomotionsschwingung).
Die Meßgrößen werden, wenn nicht anders angegeben in Mikrogefäßen mit Durchmessern ≤ 40 µm bestimmt. Die Messungen der mikrozirkulatorischen Merkmale werden am Mesenterium des Interstinum tenue und der Schleimhaut des Intestinum tenue selbst in kompletten, jeweils identischen Segmenten vorgenommen. Hierzu wird am narkotisierten und thermostatierten Tier das Intestinum operativ exponiert und in eine korperkernwarm thermostatierte Bad-Lösung (Äquivalent zur Intraperitonealflüssigkeit) ausgelagert.
Die Darstellung des mikrovaskulären Netzwerkes erfolgt mithilfe einer intravitalmikroskopischen Untersuchungseinheit im kombinierten Auflicht-DurchlichtVerfahren am unverletzten Organ mit computergestützer Bildbearbeitung und -verarbeitung. Die vitalmikroskopischen Befunderhebungen werden unter konstanten makrozirkulatorischen Randbedingungen vorgenommen.
Vor der intravitalmikroskopischen Meßwerterfassung wird jeweils eine orientierende Übersicht über das komplette mikrovaskuläre Netzwerk des gesamten Mesenterial- bzw. Darmsegmentes gewonnen - einschließlich seiner weiteren Zu- und Abflüsse, Verzweigungsgeometrie und -hierarchie und funktionellen Gefäßidentifikation. Die anatomische Gefäßidentifikation im gefärbten histologischen Präparat erfolgt nach Versuchsende im Rahmen der abschliessenden histopathologischen Untersuchung aller Versuchstiere. Die Aufnahme der Daten erfolgt üblicherweise in einem Intervall von 5 min. vor der Kontrastmittelgabe bis 10 min. nach der Kontrastmittelgabe jede Minute.

### Ergebnisse:

Die Anzahl blutzellperfundierter Knotenpunkte nimmt durch Gabe von Iloprost schneller zu.
Der Anteil aggregierter Erythrozyten an der Gesamt-Blutzellzahl im Netzwerk nimmt nach Gabe von Iloprost schneller ab.

Die Anzahl an der Venoleninnenwand adhärierender Blutzellen nimmt durch Iloprost ab.

In der nachfolgenden Tabelle sind Meßergebnisse zu oben beschriebenen Refunden im mikrovaskulären Netzwerk der Mucosa des Intestinum tenue angegeben. Eine Tiergruppe bekam einen Diatrizoat-Bolus injiziert (Spalte 1), eine zweite Gruppe erhielt eine intravenöse Applikation von 0,8 µg/kg Iloprost 5 min nach Injektion des Diatrizoat-Bolus (Spalte 2).
x bezeichnet den Mittelwert, UG die Untergrenze und OG die Obergrenze des Konfidenzintervalls.

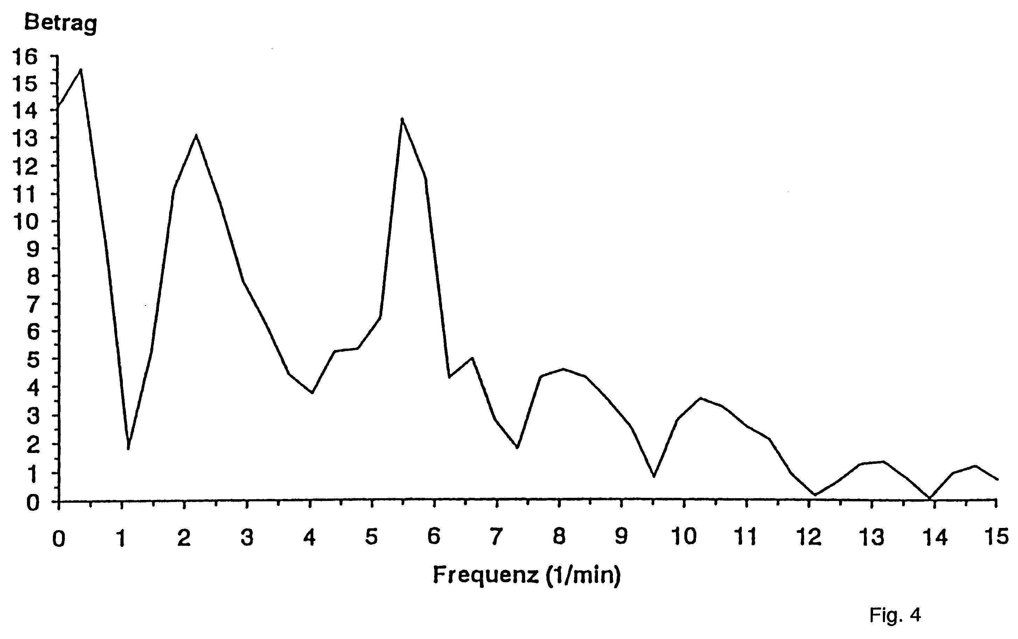

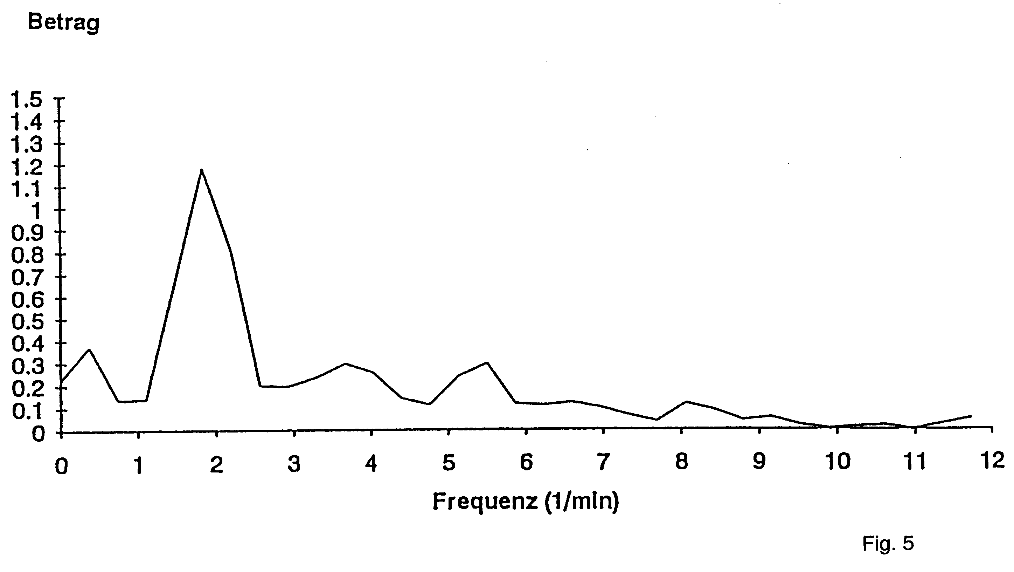

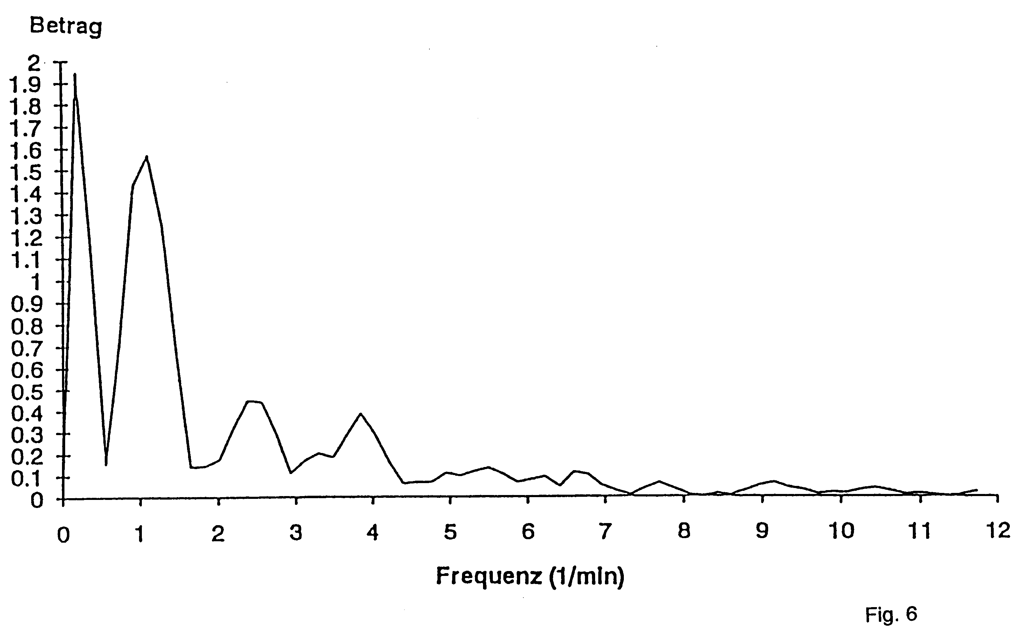

Das Frequenzspektrum der arteriolären Vasomotion weint durch Iloprost-Gabe geringere Abweichungen vom Normalzustand auf als bei ausschließlicher Applikation von Kontrastmitteln (siehe Figur 4-6).
Fig. 4 zeigt das Frequenzspektrum eines Kontrolltieres;
Fig. 5 zeigt das Frequenzspektrum eines Tieres in der 7. Minute nach intravenöser Applikation von Diatrizoat und
Fig. 6 zeigt das Frequenzspektrum eines Tieres, dem 5 min nach intravenöser Applikation von Diatrizoat auf gleichem Injektionsweg Iloprost mit einer Dosis von 0,8 µg/kg verabfolgt wurde, in der 7, Minute nach Kontrastmittelgabe, d.h. in der 2. Minute nach Iloprostinjektion.

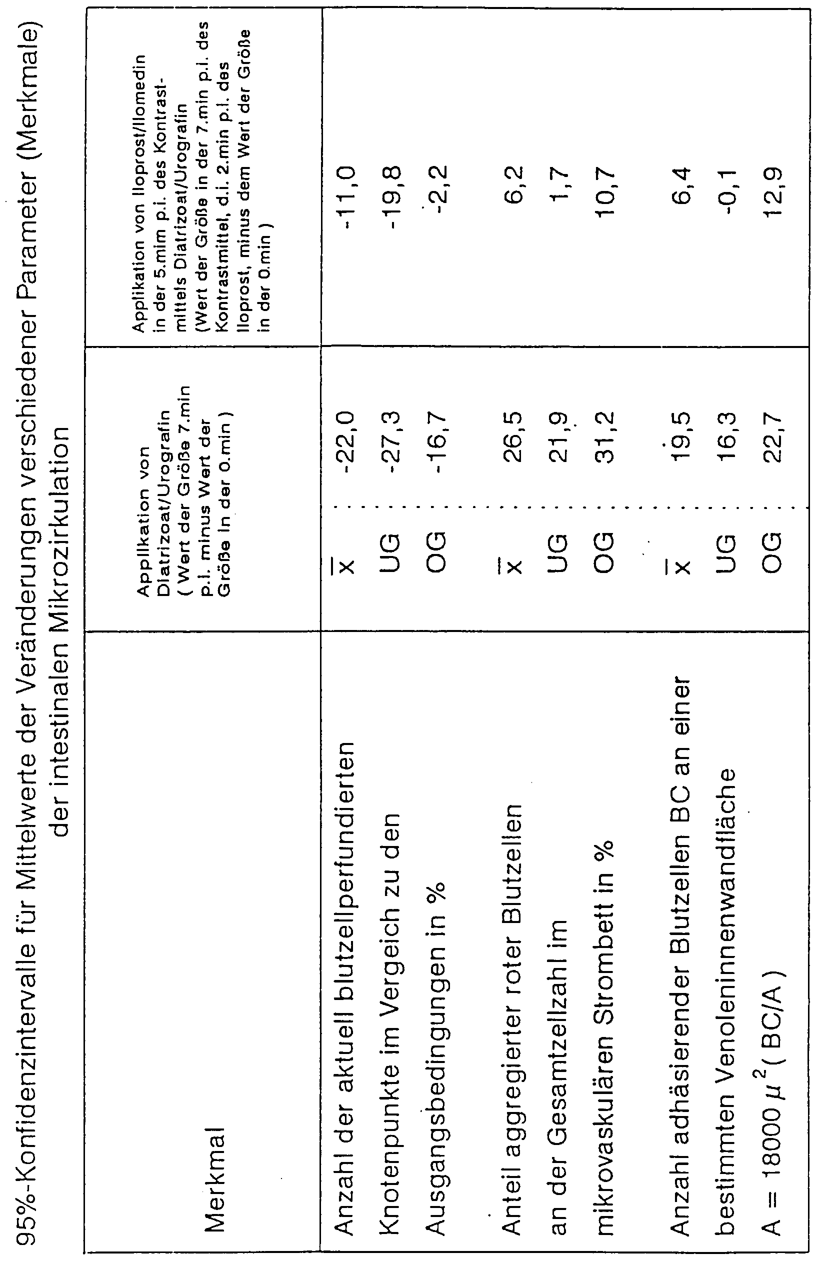

### Beispiel 3

An narkotisierten adulten Ratten [Körpermasse X=304,7g (S=35,4g)] des WistarStammes (Narkose mit Urethan-Chloralose-Gemisch - i.p., im. - , 10% Urethan in isotonischer NaCl-Lösung, 2% α-Chloralose in isotonischer NaCl-Lösung: 0,6 ml/ 100g Körpermasse als Initialdosis, 0,2 ml/100g Körpermasse als Erhaltungsdosis bei Bedarf) wird ein Mikrokatheder zur intravasalen Injektion des Kontrastmittels entweder in der A. carotis comm. bis zum Aortenbogen vorgeschoben (intraarterielle Injektion) oder in die V. femoralis eingeführt (intravenöse Injektion). Es erfolgt einmalige Injektion eines körperwarmen Bolus Diatrizoat/Urografin mit 600 mg J/kg Körpermasse. Die Injektionszeit beträgt 30s. Die Applikation von Iloprost erfolgt gleichzeitig mit der Kontrastmittelgabe, bei intravasaler Applikation auf demselben Injektionsweg wie das Kontrastmittel oder bei topischer Applikation via Intestinum/Mesenterium. Die Iloprost Dosis beträgt 1,6-0,0004 µg Iloprost/kg Körpermasse in 2,0 ml Lösung/kg Körpermasse. Bei topischer Applikation werden 3 Tropfen dieser Lösung verwendet. Als Vergleichssubstanz wird physiologische Kochsalzlösung mit entsprechendem Volumen appliziert. Aufgrund der geringen Unterschiede der Körpermassen der Versuchstiere kann bei allen Versuchstieren ein nahezu gleiches Bolusvolumen von ca. 1 ml appliziert werden, Die Zahl der Versuchstiere beträgt 10 je Behandlungsgruppe.

Folgende Meßgrößen (Merkmale) werden bestimmt:
- Anzahl der aktuell zellperfundierten Knotenpunkte im mikrovaskulären Netzwerk ( %, prozentuale Änderung der Anzahl im Vergleich zu den Ausgangsbedingungen ):
- Anzahl aggregierter Erythrozyten im mikrovaskulären Strombett ( %, prozentualer Anteil aggregierter Erythrozyten an der Gesamtzellzahl im beobachteten Strombett; als Erythrozytenaggregation wird ein längeres Aneinanderhaften von mindestens 2 bis 3 Erythrozyten beobachtet, wobei die unterschiedlichen Arten der Aggregation nicht differenziert werden );
- Anzahl adhärierender Blutzellen an der Venoleninnenwand ( BZ/A; Anzahl der Blutzellen, die länger als 5 sec am Venolenendothel anhaften - bezogen auf eine definierte Venoleninnenwandfläche A 18000µ², die bei einem Venolendurchmesser dᵥ = 40µm durch eine axiale Venolenlänge lᵥ = 140µ gegeben ist).

Arterioläre Vasomotion (Bestimmung des Innendurchmessers einer Arteriole mit einem Durchmesser von ca. 45 µm an einem bestimmten Ort zu jeder Sekunde im ca. 15 minütigen Beobachtungsintervall: Ermittlung des Ampliduden-Frequenz-Spektrums trums der Vasomotionsschwingung).
Die Meßgrößen werden, wenn nicht anders angegeben in Mikrogefäßen mit Durchmessern ≤ 40 µm bestimmt. Die Messungen der mikrozirkulatorischen Merkmale wird am Mesenterium des Interstinum tenue und der Schleimhaut des Intestinum tenue selbst in kompletten, jeweils identischen Segmenten vorgenommen. Hierzu wird am narkotisierten und thermostatierten Tier das Intestinum operativ exponiert und in eine korperkernwarm thermostatierte Bad-Lösung (Äquivalent zur Intraperitonealflussigkeit) ausgelagert.
Die Darstellung des mikrovaskulären Netzwerkes erfolgt mithilfe einer intravitalmikroskopischen Untersuchungseinheit im kombinierten Auflicht-Durchlichtverfahren am unverletzten Organ mit computergestützer Bildbearbeitung und -verarbeitung. Die vitalmikroskopischen Befunderhebungen werden unter konstanten makrozirkulatorischen Randbedingungen vorgenommen.
Vor der intravitalmikroskopischen Meßwerterfassung wird jeweils eine orientierende Übersicht über das komplette mikrovaskuläre Netzwerk des gesamten Mesenterial- bzw. Darmsegmentes gewonnen - einschließlich seiner weiteren Zu- und Abflüsse, Verzweigungsgeometrie und -hierarchie und funktionellen Gefäßidentifikation. Die anatomische Gefäßidentifikation im gefärbten histologischen Präparat erfolgt nach Versuchsende im Rahmen der abschliessenden histopathologischen Untersuchung aller Versuchstiere. Die Aufnahme der Daten erfolgt üblicherweise in einem Intervall von 5 min. vor der Kontrastmittelgabe bis 10 min. nach der Kontrastmittelgabe jede Minute.

### Ergebnisse:

Die Anzahl blutzellperfundierter Knotenpunkte nimmt durch Gabe von Iloprost schneller zu.
Der Anteil aggregierter Erythrozyten an der Gesamt-Blutzellzahl im Netzwerk nimmt nach Gabe von Iloprost schneller ab.

Die Anzahl an der Venoleninnenwand adhärierend Blutzellen nimmt durch Iloprost ab.

In der nachfolgenden Tabelle sind Meßergebnisse zu oben beschriebenen Befunden im mikrovaskulären Netzwerk der Mucosa des Intestinum tenue angegeben. Eine Tiergruppe bekam einen Diatrizoat-Bolus injiziert (Spalte 1), eine zweite Gruppe erhielt eine intravenöse Applikation von 0,8 µg/kg Iloprost gleichzeitig mit der Injektion des Diatrizoat-Bolus (Spalte 2), x bezeichnet den Mittelwert, UG die Untergrenze und OG die Obergrenze des Konfidenzintervalls.

Das Frequenzspektrum der arteriolären Vasomotion weist durch Iloprost-Gabe geringere Abweichungen vom Normalzustand auf als bei ausschließlicher Applikation vor Kontrastmitteln.

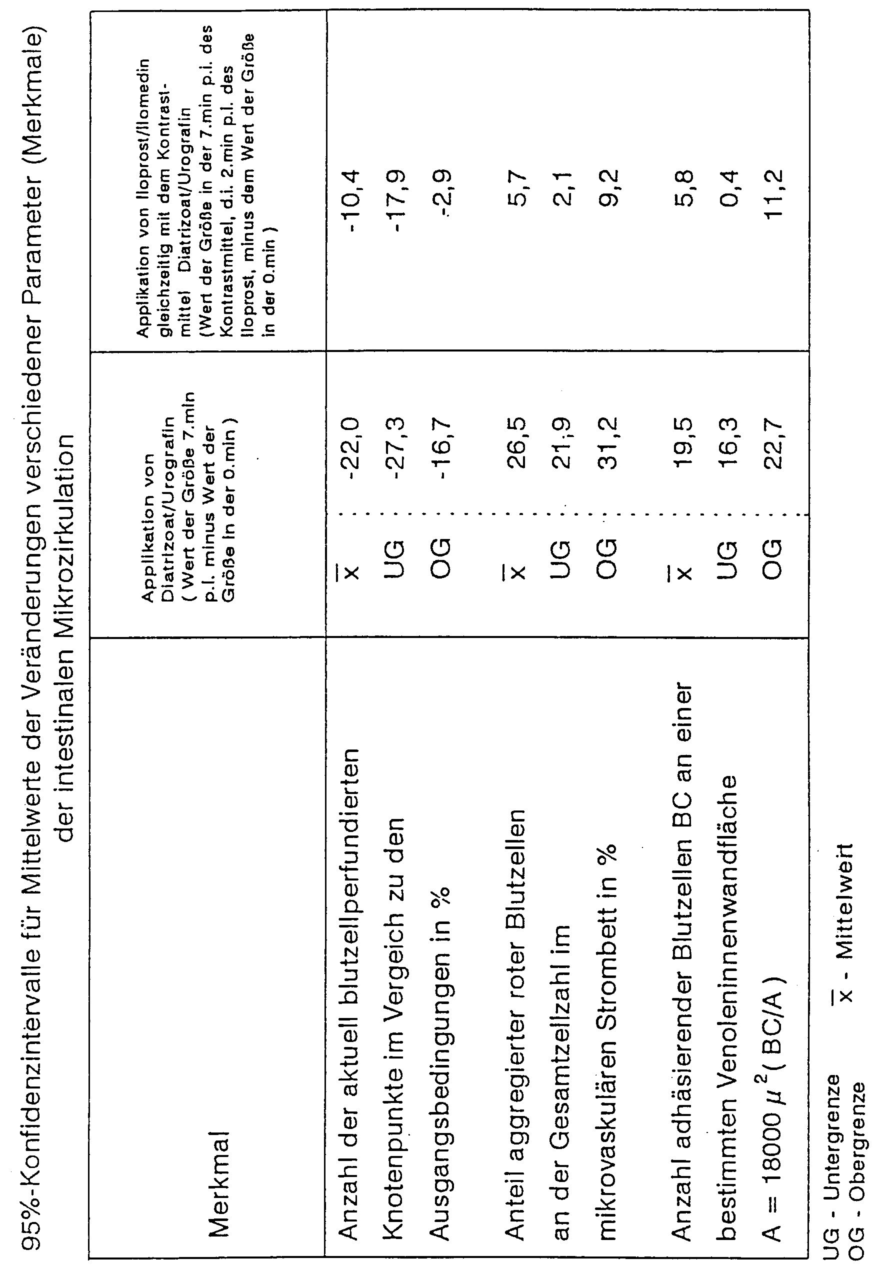

## Patentansprüche

1. Verwendung eines Prostacyclinderivates oder des entsprechenden β-Cyclodextrin-Clathrates oder der mit Liposomen verkapselten Form zur Herstellung eines Arznemittels zur Verhinderung oder Behandlung von Störungen der Mikrozirkulation bei Gabe von Röntgen-, NMR- oder Ultraschallkontrastmitteln.

2. Verwendung der Prostacyclinderivate der allgemeinen Formel I 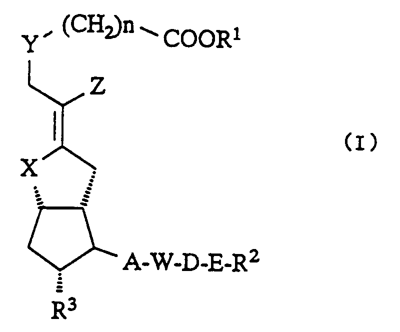 worin
R¹ Wasserstoff oder ein C₁-C₄-Alkylrest,
n 0 bis 3,
X,Y unabhängig voneinander eine -CH₂-Gruppe oder ein Sauerstoffatom,
Z Wasserstoff, Fluor oder CN,
A eine trans -CH=CH- oder eine -C=C-Gruppe,
W eine freie oder an der Hydroxygruppe funktionell abgewandelte Hydroxymethylengruppe, wobei die Hydroxygruppe α- oder β-ständig sein kann,
D eine gradkettige oder verzweigte gesättigte C₁-C₅-Alkylengruppe
E eine -C=C-Gruppe
R² eine C₁-C₂-Alkylgruppe
R³ eine freie oder funktionell abgewandelte Hydroxygruppe bedeuten, und falls R¹ Wasserstoff bedeutet,
deren Salze mit physiologisch verträglichen Rasen, sowie deren α, β- oder γ-Cyclodextrin-Clathrate sowie deren mit Liposomen verkapselte Form oder Ataprost, Beraprost, BW-15AU, Ciprosten, CS 570, FCE 22509, Naxaprosten, RS-93427, SC 39902 oder Taprosten zur Herstellung eines Arzneimittels zur Verhinderung oder Behandlung von Störungen der Mikrozirkulation bei der Gabe von Röntgen-, Ultraschall- oder NMR-Kontrastmitteln.

3. Verwendung eines Prostacyclinderivates der allgemeinen Formel I 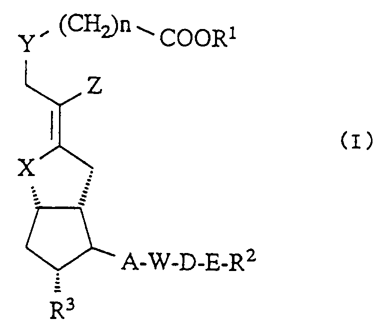 worin
R¹ Wasserstoff oder ein C₁-C₄-Alkylrest,
n 0 bis 3,
X,Y unabhängig voneinander eine -CH₂-Gruppe oder ein Sauerstoffatom,
Z Wasserstoff, Fluor oder CN,
A eine trans -CH=CH- oder eine -C=C-Gruppe,
W eine freie oder an der Hydroxygruppe funktionell abgewandelte Hydroxymethylengruppe, wobei die Hydroxygruppe α- oder β-ständig sein kann,
D eine gradkettige oder verzweigte gesättigte C₁-C₅-Alkylengruppe
E eine -C=C-Gruppe
R² eine C₁-C₂-Alkylgruppe
R³ eine freie oder funktionell abgewandelte Hydroxygruppe bedeuten, und falls R¹ Wasserstoff bedeutet,
deren Salze mit physiologisch verträglichen Basen, sowie deren α, β- oder γ-Cyclodextrin-Clathrate sowie deren mit Liposomen verkapselte Form zur Herstellung eines Arzneimittels zur Verhinderung oder Behandlung von Störungen der Mikrozirkulation bei der Gabe von Röntgen-, Ultraschall- oder NMR-Kontrastmitteln.

4. Verwendung eines der Prostacyclinderivate Ataprost, Beraprost, BW-15AU, Ciprosten, CS 570, FCE 22509, Naxaprosten, RS-93427, SC 39902 oder Taprosten zur Herstellung eines Arzneimittels zur Verhinderung oder Behandlung von Störungen der Mikrozirkulation bei der Gabe von Röntgen-, NMR- oder Ultraschall-Kontrastmitteln.

5. Verwendung von Iloprost oder Iloprost-Clathrat nach Anspruch 3.

6. Verwendung von Cicaprost oder Cicaprost-Clathrat nach Anspruch 3.

7. Verwendung von Eptaloprost oder Eptaloprost-Clathrat nach Anspruch 3.

8. Verwendung von 5-[(E)-(1S,5S,6S,7R)-7-Hydroxy-6-[(3S,4S]-3-hydroxy-4-methyl-1,6-nonadiinyl]-bicyclo[3.3,0]-oct-3-yliden]-pentansäure oder dem entsprechenden Clathrat nach Anspruch 3.

9. Verwendung von 5-[(E)-(1S,5S,6S,7R)-7-Hydroxy-6-[(3S,4S)-3-hydroxy-4-methyl-1,6-nonadiinyl]-bicyclo[3.3.0]-oct-3-yliden]-5-fluoro-3-oxa-pentansäure oder dem entsprechenden Clathrat nach Anspruch 3.

## Claims

1. Use of a prostacyclin derivative or of the corresponding β-cyclodextrin clathrate or of the liposome-encapsulated form for the preparation of a medicament for the prevention or treatment of disturbances of the microcirculation on administration of X-ray, NMR or ultrasound contrast media.

2. Use of the prostacyclin derivatives of the general formula I 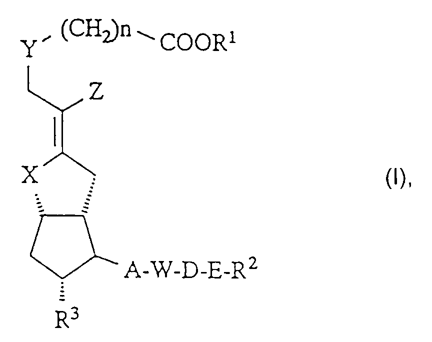 wherein
R¹ represents hydrogen or a C₁-C₄alkyl radical,
n is from 0 to 3,
X and Y each independently of the other represents a -CH₂- group or an oxygen atom,
Z represents hydrogen, fluorine or CN,
A represents a trans -CH=CH- group or a -C=C- group,
W represents a hydroxymethylene group, which is free or functionally modified at the hydroxy group, it being possible for the hydroxy group to be in the α- or β- position,
D represents a straight-chained or branched, saturated C₁-C₅alkylene group,
E represents a -C=C- group,
R² represents a C₁-C₂alkyl group,
R³ represents a free or functionally modified hydroxy group, and,
if R¹ represents hydrogen,
of salts thereof with physiologically tolerable bases, and of α-, β- or γ-cyclodextrin clathrates thereof and of the liposome-encapsulated form thereof, or of ataprost, beraprost, BW-15AU, ciprostene, CS 570, FCE 22509, naxaprostene, RS-93427, SC 39902 or taprostene, for the preparation of a medicament for the prevention or treatment of disturbances of the microcirculation on administration of X-ray, ultrasound or NMR contrast media.

3. Use of a prostacyclin derivative of the general formula I 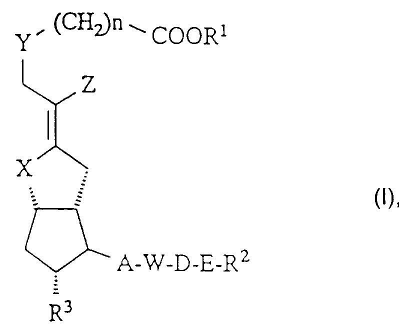 wherein
R¹ represents hydrogen or a C₁-C₄alkyl radical,
n is from 0 to 3,
X and Y each independently of the other represents a -CH₂- group or an oxygen atom,
Z represents hydrogen, fluorine or CN,
A represents a trans -CH=CH- group or a -C=C- group,
W represents a hydroxymethylene group, which is free or functionally modified at the hydroxy group, it being possible for the hydroxy group to be in the α- or β- position,
D represents a straight-chained or branched, saturated C₁-C₅alkylene group,
E represents a -C=C- group,
R² represents a C₁-C₂alkyl group,
R³ represents a free or functionally modified hydroxy group, and,
if R¹ represents hydrogen,
of salts thereof with physiologically tolerable bases, and of α-, β- or γ-cyclodextrin clathrates thereof and of the liposome-encapsulated form thereof, for the preparation of a medicament for the prevention or treatment of disturbances of the microcirculation on administration of X-ray, ultrasound or NMR contrast media.

4. Use of one of the prostacyclin derivatives ataprost, beraprost, BW-15AU, ciprostene, CS 570, FCE 22509, naxaprostene, RS-93427, SC 39902 or taprostene for the preparation of a medicament for the prevention or treatment of disturbances of the microcirculation on administration of X-ray, NMR or ultrasound contrast media.

5. Use of iloprost or iloprost clathrate according to claim 3.

6. Use of cicaprost or cicaprost clathrate according to claim 3.

7. Use of eptaloprost or eptaloprost clathrate according to claim 3.

8. Use of 5-[(E)-(1S,5S,6S,7R)-7-hydroxy-6-[(3S,4S)-3-hydroxy-4-methyl-1,6-nonadiynyl]-bicyclo[3.3.0]oct-3-ylidene]-pentanoic acid or the corresponding clathrate according to claim 3.

9. Use of 5-[(E)-(1S,5S,6S,7R)-7-hydroxy-6-[(3S,4S)-3-hydroxy-4-methyl-1,6-nonadiynyl]-bicyclo[3.3.0]oct-3-ylidene]-5-fluoro-3-oxapentanoic acid or the corresponding clathrate according to claim 3.

## Revendications

1. Utilisation d'un dérivé de prostacycline ou du clathrate de β-cyclodextrine correspondant, ou de la forme encapsulée dans des liposomes, pour la préparation d'un médicament pour prévenir ou traiter les troubles de la microcirculation lors de l'administration de produits de contraste pour le diagnostic aux rayons X, par la RMN ou aux ultrasons.

2. Utilisation de dérivés de prostacycline de formule générale I 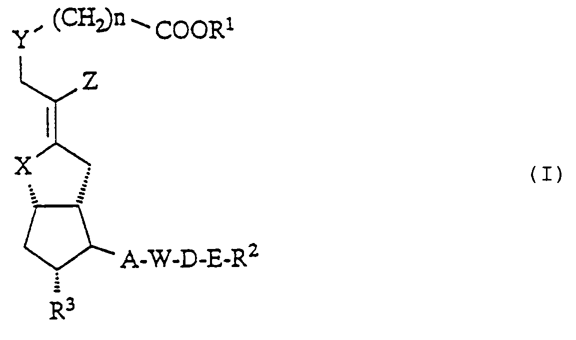 dans laquelle
R¹ représente l'hydrogène ou un reste alkyle en C₁-C₄,
n va de 0 à 3,
X, Y, indépendamment l'un de l'autre, représentent un groupe -CH₂- ou un atome d'oxygène,
Z représente l'hydrogène, le fluor ou CN,
A représente un groupe trans-CH=CH- ou un groupe -C=C-,
W représente un groupe hydroxyméthylène libre ou fonctionnellement modifié sur le groupe hydroxy, le groupe hydroxy pouvant être en position α ou β,
D représente un groupe alkylène en C₁-C₅, linéaire ou ramifié, saturé,
E représente un groupe -C=C-
R² représente un groupe alkyle en C₁-C₂,
R³ représente un groupe hydroxy libre ou fonctionnellement modifié, et dans le cas où R¹ représente l'hydrogène,
leurs sels avec des bases physiologiquement tolérés, ainsi que leurs clathrates d'α-, de β- ou de γ-cyclodextrine, ainsi que leur forme encapsulée dans des liposomes, ou l'Ataprost, le Beraprost, BW-15AU, le Ciprosten, CS 570, FCE 22509, le Naxaprosten, RS-93427, SC 39902 ou le Taprosten pour la préparation d'un médicament pour prévenir ou traiter les troubles de la microcirculation lors de l'administration des produits de contraste pour le diagnostic aux rayons X, aux ultrasons ou par la RMN.

3. Utilisation d'un dérivé de prostacycline de formule générale I 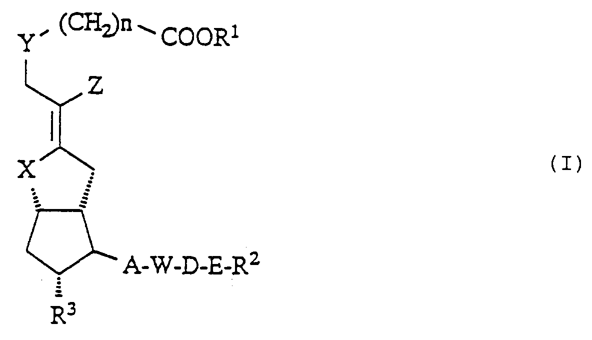 dans laquelle
R¹ représente l'hydrogène ou un reste alkyle en C₁-C₄,
n va de 0 à 3,
X, Y, indépendamment l'un de l'autre, représentent un groupe -CH₂- ou un atome d'oxygène,
Z représente l'hydrogène, le fluor ou CN,
A représente un groupe trans-CH=CH- ou un groupe -C=C-,
W représente un groupe hydroxyméthylène libre ou fonctionnellement modifié sur le groupe hydroxy, le groupe pouvant être en position α ou β,
D représente un groupe alkylène en C₁-C₅, linéaire ou ramifié, saturé,
E représente un groupe -C=C-
R² représente un groupe alkyle en C₁-C₂,
R³ représente un groupe hydroxy libre ou fonctionnellement modifié, et lorsque R¹ représente l'hydrogène,
leurs sels avec des bases physiologiquement tolérés, ainsi que leurs clathrates d'α-, de β- ou de γ-cyclodextrine, ainsi que leur forme encapsulée dans des liposomes, ou l'Ataprost, le Beraprost, BW-15AU, le Ciprosten, CS 570, FCE 22509, le Naxaprosten, RS-93427, SC 39902 ou le Taprosten pour la préparation d'un médicament pour prévenir ou traiter les troubles de la microcirculation lors de l'administration des produits de contraste pour le diagnostic aux rayons X, aux ultrasons ou par la RMN.

4. Utilisation d'un dérivé de prostacycline Ataprost, Beraprost, BW-15AU, Ciprosten, CS 570, FCE 22509, Naxaprosten, RS-93427, SC 39902 ou Taprosten pour la préparation de médicaments pour empêcher ou traiter des troubles de la microcirculation lors de l'administration des produits de contraste pour les examens aux rayons X, aux ultrasons ou par la RMN.

5. Utilisation de l'iloprost ou du clathrate d'iloprost selon la revendication 3.

6. Utilisation du cicaprost ou du clathrate de cicaprost selon la revendication 3.

7. Utilisation de l'eptaloprost ou du clathrate d'eptaloprost selon la revendication 3.

8. Utilisation de l'acide 5-[(E)-(1S,5S,6S,7R)-7-hydroxy-6-[(3S,4S)-3-hydroxy-4-méthyl-1,6-nonadiinyl]-bicyclo[3.3.0]-oct-3-ylidène]-pentanoïque ou du clathrate correspondant selon la revendication 3.

9. Utilisation de l'acide 5-[(E)-(1S,5S,6S,7R)-7-hydroxy-6-[(3S,4S)-3-hydroxy-4-méthyl-1,6-nonadiinyl]-bicyclo[3.3.0]-oct-3-ylidène]-5-fluoro-3-oxa-pentanoïque ou du clathrate correspondant selon la revendication 3.
